# EUROPÄISCHE PATENTSCHRIFT

(11) **EP 3 278 836 B1**
(45) Veröffentlichungstag und Bekanntmachung des Hinweises auf die Patenterteilung: **16.12.2020**
(21) Anmeldenummer: 17183988.9
(22) Anmeldetag: 31.07.2017
(51) Int. Cl.: A61N 1/375, A61N 1/362, A61N 1/36, A61N 1/372

(54) **ELEKTROMEDIZINISCHES IMPLANTAT MIT EINER ELEKTRISCHEN DURCHFÜHRUNG**
ELECTROMEDICAL IMPLANT WITH AN ELECTRIC FEEDTHROUGH
IMPLANT ÉLECTROMÉDICAL COMPRENANT UN PASSAGE ÉLECTRIQUE

(30) Priorität: 01.08.2016 DE 102016114155
(43) Veröffentlichungstag der Anmeldung: 07.02.2018
(73) Patentinhaber: BIOTRONIK SE & Co. KG, 12359 Berlin (DE)
(72) Erfinder: Starke, Marcel, 15732 Eichwalde (DE); Romberg, Jan, 12347 Berlin (DE)
(74) Vertreter: Keck, Hans-Georg

(56) Entgegenhaltungen:
- US-A1- 2011 077 708
- US-A1- 2012 290 021
- US-A1- 2014 058 240
- US-A1- 2014 100 627
- US-A1- 2015 073 507
- US-A1- 2015 321 016

## Beschreibung

Die Erfindung ist gerichtet an elektromedizinische Implantate zur Messung sowie zur Abgabe von elektrischen Pulsen oder auch z. B. als implantierbare Zentraleinheit zur drahtlosen Signalübertragung zwischen mehreren Implantaten und an den optimierten Aufbau derartiger Implantate.

Elektromedizinische Implantate zur Messung von elektrischen Potentialen sowie zur Abgabe von elektrischen Pulsen werden seit Jahrzehnten im menschlichen und tierischen Körper zur Messung von elektrischen Potentialen eingesetzt, die durch Nervenaktivität hervorgerufen werden. Auch seit langem bekannt sind Implantate, die zur elektrischen Stimulation von Nervenstrukturen eingesetzt werden. Typische Indikation für den Bedarf eines solchen Implantats ist eine gestörte und/oder nicht bedarfsgerechte körpereigene (intrinsische) Nervenaktivität bzw. Erregungsleitung. Dabei existieren verschiedene Mess- und/oder Stimulationsgeräte, die sich je nach technischen Eigenschaften sowie nach Einsatzbereich des Nervensystems in Form und Ausführung unterscheiden.

Systeme für die Rückenmarksstimulation werden beispielsweise eingesetzt, wenn Nervenstrukturen für die Funktionalität der Extremitäten geschädigt wurden, dann in der Regel zur Schmerztherapie oder zur Unterstützung der Rehabilitation. Derartige Implantate bestehen z. B. aus einem Geräteaggregat und verschiedenen Elektrodenleitern. Das Geräteaggregat verfügt über die Gerätelektronik, welche beispielsweise elektrische Schaltungen für Messungen und Analyse der elektrischen Potentiale sowie für Steuerung der elektrischen Stimulation beinhalten. Auch beinhaltet das Geräteaggregat typischerweise eine Energiequelle für das Implantat, wie eine Batterie, sowie elektrische Anschlüsse für die Elektrodenleiter. Bekannte Elektrodenleiter für derartige Einsatzzwecke besitzen einen länglichen Leiterkörper, eine oder mehrere Elektroden am distalen Ende des Leiters sowie einen elektrischen Kontakt am proximalen Ende des Leiters, mit dem sie an die elektrischen Anschlüsse des Geräteaggregats angeschlossen werden können. Beim Implantationsvorgang wird das Geräteaggregat subkutan an einer geeigneten Stelle unter der Haut des Patienten eingesetzt, während die Elektrodenleiter an für Messungen und Stimulationen des Nervengewebes geeignete Orte implantiert und an das Geräteaggregat angeschlossen werden.

Einen ähnlichen Aufbau wie das beschriebene Rückenmarksstimulationssystem besitzen bekannte Systeme für die Stimulation des Herzens. So bestehen gängige Herzschrittmachersysteme, implantierbare kardiale Defibrillatoren oder Systeme zur Resynchronisation des Herzens (Cardiac Rhythm Therapy, CRT Systeme) auch typischerweise aus Geräteaggregat und Elektrodenleiter. Für die Messung und Stimulation der rechten Herzhälfte werden Elektrodenleiter über die obere Hohlvene in den rechten Vorhof und rechten Ventrikel des Herzens vorgeschoben und am gewünschten Implantationsort verankert. Elektrodenleiter für die Messung und Stimulation der linken Herzhälfte werden typischerweise in einer Abzweigung des Koronarsinus platziert. Das Geräteaggregat eines solchen Herzstimulators wird im Schlüsselbeinbereich des Patienten subkutan eingebettet und mit dem Elektrodenleiter verbunden.

Es sind Herzschrittmachersysteme im Stand der Technik bekannt, die nicht über oben beschriebene Elektrodenleiter verfügen. Solche Herzschrittmachersysteme werden auch als 'leadless' Systeme bezeichnet, genauer ,leadless pacemaker'. Derartige Systeme verfügen über ein Geräteaggregat, bei welchem die Elektroden zur Messung und Stimulation am Gehäuse lokalisiert sind. Das Geräteaggregat wird über ein Kathetersystem direkt im Vorhof oder im Ventrikel des Herzens implantiert und dort fixiert.

Weiterhin sind auch implantierbare reine Überwachungsgeräte für das Herz bekannt. Diese werden subkutan platziert und verfügen nicht über Elektrodenleiter, die intrakardial implantiert werden. Solche Geräte weisen Messelektroden am Gerätegehäuse auf, über die elektrische Potentiale aufgenommen werden, um beispielsweise Herzsignale herauszufiltern.

Genauer gesagt sind bekannte Aufbauten von besagten'leadless' Systemen und reinen Überwachungsgeräten dergestalt, dass sie über ein Gerätegehäuse verfügen, in welchem Geräteelektronik und Energieversorgung beherbergt sind. Innerhalb des Gehäuses sind Geräteelektronik und Energieversorgung über eine elektrische Durchführung miteinander elektrisch verbunden. Des Weiteren sieht ein solcher typischer Aufbau vor, dass die Geräteelektronik über eine weitere elektrische Durchführung mit einer Messelektrode verbunden ist. Dabei ist das Gehäuse elektrisch gekoppelt mit dem zweiten Elektrodenpol für die elektrische Messung und/oder elektrische Stimulation und dient somit als Gegenpol zur Messelektrode.

Beispielsweise wird in US 2015/0073507 A1 ein implantierbares medizinisches Gerät vorgeschlagen, wobei das medizinische Gerät über mindestens zwei Elektroden verfügt, die an das Gerätegehäuse gekoppelt sind.

US 2015/0321016 A1 beschreibt ein medizinisches Gerätesystem das dazu ausgelegt ist, über ein erstes Gerät ein physiologisches Signal zu messen, wobei das erste Gerät ein Kontrollsignal als Antwort auf das gemessene physiologische Signal zu generiert.

US 2014/0100627 A1 offenbart ein "leadless" intra-kardiales medizinisches Gerät, welches eine integrierte, LC-resonante Drucksensor-Schaltung umfasst.

US 2012/0290021 A1 beschreibt eine Batteriedurchführung, welche einen Ring umfasst, wobei der Ring eine Passage aufweist, die sich von einer ersten Seite zu einer zweiten Seite erstreckt.

US 2015/0073507 A1 lehrt ein implantierbares medizinisches Gerät mit zwei Elektroden, die an das Gerätegehäuse gekoppelt sind.

US 2011/0077708 A1 beschreibt einen implantierbaren batteriebetriebenen 'leadless' Schrittmacher. Der Schrittmacher kann u.a. in Modi einer MRT (Magnetische Resonanztomographie) Umgebung sicher betrieben werden.

US 2014/0058240 A1 offenbart ein implantierbares medizinisches Gerät, das einen Elektrode aufweist, die eine einschnappende und einrastende Befestigungsbereich bildet.

Die bislang bekannten ,leadless' Systeme, implantierbare Überwachungsgeräte, sowie allgemeine implantierbare elektromedizinische Geräte und insbesondere letztere ohne Elektrodenleitungen besitzen einen relativ komplexen Geräteaufbau z.B. mit elektrischen Durchführungen und separaten Antennen.

Für beschriebene ,leadless' Systeme, implantierbare Überwachungsgeräte, sowie allgemein für implantierbare elektromedizinische Geräte und insbesondere für letztere ohne Elektrodenleitungen ist eine Miniaturisierung des Geräteaufbaus bzw. Gerätegehäuses vorteilhaft.

Es ist deshalb Aufgabe der vorliegenden Erfindung, ein neuartiges implantierbares elektromedizinisches Gerät bereitzustellen, das in seinen Dimensionen und im Volumen kleiner ist als bisherige bekannte implantierbare Geräte vergleichbarer Art.

Eine weitere Aufgabe der vorliegenden Erfindung liegt darin, ein neuartiges implantierbares elektromedizinisches Gerät bereitzustellen, das einen neuartigen Geräteaufbau aufweist, welcher einfach und kostengünstig herzustellen ist.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Gemäß der vorliegenden Erfindung wird ein elektromedizinisches Implantat vorgeschlagen, welches ein Gehäuse umfasst, wobei das Gehäuse drei Gehäusesegmente A, B und C, aufweist. Gehäusesegment A ist mit ist mit Gehäusesegment B verbunden, und Gehäusesegment B ist mit Gehäusesegment C verbunden, sodass Gehäusesegment B zwischen den Gehäusesegmenten A und C angeordnet ist. Dabei bestehen Gehäusesegment A sowie Gehäusesegment C mindestens teilweise aus elektrisch leitendem Material. Des Weiteren umfasst das erfindungsgemäße elektromedizinische Implantat ein Elektronikmodul, das innerhalb des Gehäusesegments A angeordnet ist und eine elektrische Schaltung zur Aufnahme und Weiterverarbeitung von elektrischen Signalen umfasst, sowie mindestens einen ersten und einen zweiten elektrischen Leiter zur Messung von elektrischen Potentialen und/oder Abgabe von elektrischen Pulsen, wobei jeder Leiter jeweils einen Leiteranfang und ein Leiterende aufweist und jeder Leiteranfang mit der elektrischen Schaltung verbunden ist. Auch umfasst das erfindungsgemäße elektromedizinische Implantat ein Batteriemodul, das im Inneren des Gehäusesegments C angeordnet ist, oder wobei das Batteriemodul ein Batteriegehäuse aufweist, welches durch Gehäusesegment C gebildet wird. Dabei weist das erfindungsgemäße elektromedizinische Implantat eine elektrische Durchführung auf, wobei die elektrische Durchführung mindestens teilweise aus elektrisch isolierendem Material besteht und Gehäusesegment B durch Teile der Durchführung gebildet wird. Gemäß der Erfindung sind das Elektronikmodul und das Batteriemodul über die Durchführung elektrisch verbunden, wobei Gehäusesegment A und Gehäusesegment C durch die Durchführung voneinander elektrisch isoliert sind. Des Weiteren ist das Leiterende des ersten elektrischen Leiters mit dem aus elektrisch leitendem Material bestehenden Teil des Gehäusesegments C über die Durchführung elektrisch verbunden, und das Leiterende des zweiten elektrischen Leiters ist mit dem aus elektrisch leitendem Material bestehenden Teil des Gehäusesegments A elektrisch verbunden.

In einer bevorzugten Ausführungsform weist das erfindungsgemäße elektromedizinische Implantat eine einzige elektrische Durchführung auf.

In diesem Zusammenhang soll unter einem "Gehäusesegment" verstanden werden, dass es sich um einen Abschnitt des Gehäuses handelt. Dabei können die Gehäusesegmente A und/oder C kann aus einem einzigen Material bestehen, wie zum Beispiel aus Metall oder einem anderen dem Fachmann als zweckdienlich erscheinenden, elektrisch leitenden Material, welches vorzugsweise Biokompatibilität aufweist. Des Weiteren ist auch denkbar, dass ein solches Segment verschiedene Materialien umfasst, wie beispielsweise aus einem Materialverbund bzw. Verbundmaterial besteht. Mögliche geeignete Materialien sind biokompatible und leitfähige Metalle und Metalllegierungen wie Titan, Titanlegierungen, Edelstähle (u.a. 316L), andere Legierungen wie z.B. MP35N, leitfähige und biokompatible Kunststoffe und Kunststoff- bzw. Keramik-Verbundmaterialien. Gehäusesegment B besteht vorzugsweise zumindest teilweise aus elektrisch isolierendem Material. Geeignet für diesen Zweck wären beispielsweise Glas, Keramik, Kunststoffe bzw. Verbundmaterialien aus den genannten.

In einer bevorzugten Ausführungsform des erfindungsgemäßen elektromedizinischen Implantats besteht Gehäusesegment B aus elektrisch isolierendem Material, wobei das Gehäuse so ausgelegt ist, sodass Gehäusesegment A und Gehäusesegment C durch Gehäusesegment B voneinander elektrisch isoliert sind.

In einer bevorzugten Ausführungsform des erfindungsgemäßen elektromedizinischen Implantats sind Gehäusesegmente A und C als Gehäuseschalen ausgeführt. In diesem Zusammenhang soll unter einer "Gehäuseschale" ein geometrischer Hohlkörper mit mindestens einer Öffnung verstanden werden, welcher vorteilhaft als Teil eines Gehäuses für ein Implantat gemäß der vorliegenden Erfindung verwendet werden kann. Als vorteilhaft können Formen der Gehäuseschale angesehen werden, die nach der Implantation für einen hohen Patientenkomfort sorgen sowie Formen, die mit einem geringen Infektionsrisiko nach der Implantation einhergehen und/oder solche, die einfach zu implantieren sind und nach der Implantation fest an ihrem Implantationsort verbleiben, ohne dass sie verrutschen oder sich verdrehen. Dabei bietet es sich an, wenn die Form der Gehäuseschale eine kleine Gesamtaußenfläche relativ zum Innenvolumen aufweist. Auch sind bieten sich Formen mit wenigen Ecken und Kanten an, bzw. mit abgerundeten Ecken und Kanten. Beispielsweise kann eine Gehäuseschale als hohler Quader mit einer offenen Seitenwand oder Paraboloid ausgeführt sein.

Das Elektronikmodul des erfindungsgemäßen elektromedizinischen Implantats ist im Inneren des Gehäusesegments A angeordnet und umfasst eine elektrische Schaltung. Diese beinhaltet beispielsweise elektrische Schaltkreise für die Aufnahme und Verarbeitung von elektrischen Potentialen sowie Einheiten zur Speicherung, Signalweiterverarbeitung und Signalanalyse. Des Weiteren kann der Schaltkreis Komponenten für die Abgabe von elektrischen Pulsen sowie zur Koordination dieser Pulse beinhalten. Ebenfalls denkbar wären Schaltkreiskomponenten für die drahtlose Datenübertragung (Telemetrie) von gemessenen und gespeicherten Daten an externe Systeme oder ein externes Patientengerät. Weiterhin umfasst das Elektronikmodul mindestens einen ersten und zweiten Leiter. Die Leiter sind mit ihrem jeweiligen Leiteranfang mit der elektrischen Schaltung verbunden.

Im Zusammenhang der vorliegenden Erfindung soll unter einem "elektrischen Leiter" allgemein eine elektrische Leiterverbindung verstanden werden, mit der elektronische Elemente kontaktiert werden können. Eine solche Leiterverbindung kann beispielsweise als Leiterdraht mit Leiteranfang und Leiterende aufgeführt sein, oder als Leiterbahn auf einer Platine mit elektrischer Kontaktierungsmöglichkeit an Anfang und Ende der Leiterbahn, oder als leitender Teil einer elektrischen Durchführung ausgelegt sein, oder einer Kombination aus den genannten und weiteren, für den Fachmann zweckdienlichen Möglichkeiten.

Das Batteriemodul ist im Inneren des Gehäusesegments C angeordnet. In einer bevorzugten Ausführungsform besteht keine direkte elektrische Verbindung zwischen dem Batteriemodul und Gehäusesegment C. Unter "keine direkte elektrische Verbindung" soll im Sinne der Ausführungsform der Erfindung verstanden werden, dass Batteriemodul und Gehäusesegment C derart elektrisch isoliert sein sollen, dass keine unmittelbare elektrische Verbindung über einen elektrischen Leiter zwischen Batteriemodul und Gehäusesegment C implementiert ist. Hintergrund dafür ist, dass in einer bevorzugten Ausführungsform der Erfindung Gehäusesegment C mit der elektrischen Schaltung des Elektronikmoduls elektrisch verbunden ist und dass das elektrische Potential des aus elektrisch leitendem Material bestehenden Teils des Gehäusesegments C durch diese Verbindung bestimmt wird.

Gemäß der vorliegenden Erfindung weist das erfindungsgemäße elektromedizinische Implantat eine elektrische Durchführung auf. Im Sinne der Erfindung wird unter einer elektrischen Durchführung ein Element zur elektrischen Leitung verstanden, welches ausgelegt ist, ein elektrisches Signal von einer Umgebung in eine andere überzuführen bzw. einzukoppeln, wobei Signaleigenschaften und -Qualität so gut wie möglich erhalten bleiben sollen. Beispielsweise kann es sich dabei um die Einkopplung eines Signals in ein Gerätegehäuse, oder von einem Gehäusesegment in ein anderes Gehäusesegment handeln. Im Sinne der Erfindung besteht die elektrische Durchführung aus einem Durchführungskörper, in den elektrische Leiterelemente integriert sind bzw. durch den elektrische Leiterelemente durchführen. Dabei führen die Leiterelemente jeweils von einer ersten Außenfläche des Durchführungskörpers durch den Durchführungskörper hindurch zu einer zweiten Außenfläche des Durchführungskörpers. Die erste und die zweite Außenfläche können hierbei dieselbe Außenfläche oder unterschiedliche Außenflächen des Durchführungskörpers darstellen. Die Leiterelemente verfügen an ihren Enden über elektrische Kontakte, die an der entsprechenden Außenfläche überstehen und über diese das elektrische Signal in die Durchführung eingekoppelt werden kann. In einer Ausführungsform werden besagte elektrische Kontakte durch Anlöten am Durchführungskörper angebunden werden. Bevorzugt besteht der Durchführungskörper mindestens teilweise aus einem elektrisch isolierenden Material. In einer Ausführungsform besteht der gesamte Durchführungskörper aus einem solchen Material. Auf jeden Fall jedoch ist im Sinne der Erfindung die elektrische Durchführung bzw. der Durchführungskörper ausgelegt, Gehäusesegmente A und C elektrisch voneinander zu isolieren, sodass kein direkter Strompfad von Gehäusesegment A über die Durchführung zu Gehäusesegment C besteht. Dabei wird das Gehäusesegment B durch Teile der Durchführung gebildet, beispielsweise von einer Anzahl an Außenflächen des Durchführungskörpers. Beispielsweise kann die Gesamtheit des Gehäusesegments B durch Teile der Durchführung bzw. des Durchführungskörpers gebildet werden. Innerhalb des Gehäuses ist, wie zuvor erwähnt, das Elektronikmodul im Inneren des Gehäusesegments A angeordnet, während das Batteriemodul im Inneren des Gehäusesegments C angeordnet ist. Dabei sind das Elektronikmodul und das Batteriemodul vorzugsweise elektrisch über/durch die Durchführung miteinander verbunden. Dies kann durch zusätzliche elektrische Leiter gewährleistet werden, die das Elektronikmodul mit elektrischen Kontakten an der zum Elektronikmodul gerichteten Seite der Durchführung verbinden, sowie zusätzliche elektrische Leiter, die das Batteriemodul mit elektrischen Kontakten an der zum Batteriemodul gerichteten Seite der Durchführung verbinden.

Weiterhin ist besagter erster elektrischer Leiter, dessen Leiteranfang mit der elektrischen Schaltung verbunden ist, mit dem Leiterende mit einem Teil des Gehäusesegments C verbunden, der aus elektrisch leitendem Material besteht, wobei der erste Leiter durch die Durchführung führt. Der Strompfad der ersten Leiters führt somit von der elektrischen Schaltung durch die Durchführung zum Gehäusesegment C, der aus elektrisch leitendem Material besteht. Besagter zweiter elektrischer Leiter, dessen Leiteranfang ebenfalls mit der elektrischen Schaltung verbunden ist, ist mit dem Leiterende mit dem aus elektrisch leitendem Material bestehenden Teil des Gehäusesegments A verbunden. Gemäß der vorliegenden Erfindung besteht zwischen Gehäusesegment A bzw. C und dem Elektronikmodul bzw. dem Batteriemodul außer den elektrischen Verbindungen über den ersten und zweiten Leiter (sowie eventuell erforderliche Massepotential-Verbindungen) keine weitere elektrische Verbindung. Auf diese Weise wird ein Strompfad erzeugt, der von der elektrischen Schaltung des Elektronikmoduls zum elektrisch leitenden Teil des Gehäusesegments A führt, von dort aus zum elektrisch leitenden Teil des Gehäusesegments C und dann über den zweiten Leiter durch die Durchführung zurück zur elektrischen Schaltung. Folglich werden die zwei für die Messung bzw. Abgabe von elektrischen Pulsen erforderlichen Elektrodenpole gebildet durch den elektrisch leitenden Teil von Gehäusesegment A und Gehäusesegment C. Wie zuvor erwähnt können dabei Gehäusesegment A und/oder C auch gänzlich aus elektrisch leitendem Material bestehen.

In einer vorteilhaften Ausführung der vorliegenden Erfindung sind Gehäusesegmente A und C gegebenenfalls mittels weiterer elektrischer Leiter, welche durch die Durchführung führen, kontaktiert. Dabei ist in einer Ausführungsform denkbar, dass Gehäusesegment A bzw. C (oder der elektrisch leitende Teil des jeweiligen Segments) jeweils fest auf dem Plus- bzw. Minuspol der Batterie des Batteriemoduls liegen. Auch denkbar ist es, dass das elektrische Potential der elektrisch leitenden Teile der Gehäusesegmente A und C veränderlich (auf Englisch ,floating') gestaltet sind.

In einer Ausführungsform des erfindungsgemäßen elektromedizinischen Implantats weist das Gehäuse des Implantats eine längliche Form auf, wobei Gehäusesegmente A und C als Gehäuseschalen ausgeführt sind. Die Gehäuseschalen sind jeweils an den offenen Enden mit der Durchführung fest verbunden. Dabei handelt es sich bevorzugt um eine Löt- oder Schweißverbindung.

Gemäß einer Ausführungsform ist das erfindungsgemäße elektromedizinische Implantat ein kardiales Implantat in Form eines Sensors, Überwachungsgeräts, Schrittmachergeräts, Schrittmachergeräts mit integrierter Defibrillatorfunktion oder Schrittmachergeräts ohne intrakardiale Elektroden ausgeführt. Denkbar wären auch sämtliche implantierbare elektromedizinische Geräte, die über zwei Elektroden verfügen und für die eine Miniaturisierung des Implantats vorteilhaft ist. In einer bevorzugten Ausführung der Erfindung handelt es sich beim elektromedizinischen Implantat um ein reines kardiales Überwachungsimplantat (Monitoring-Implantat) oder um ein miniaturisiertes kardiales Schrittmacherimplantat ohne intrakardiale Elektrodenleiter oder ,leadless pacemaker' Implantat.

Gemäß einer Ausführungsform ist das erfindungsgemäße elektromedizinische Implantat ein Neurostimulationsimplantat in Form eines Rückenmarkstimulationsgeräts, Vagusnerv-Stimulationsgeräts, oder Hirnstimulationsgeräts. Auch denkbar wäre die Ausführung des elektromedizinischen Implantats als Muskelstimulationgerät.

In einer vorteilhaften Ausgestaltung der Erfindung weist das Gehäusesegment A und/oder Gehäusesegment C des vorgeschlagenen elektromedizinischen Implantats eine Beschichtung aus elektrisch isolierendem Material auf, wobei Gehäusesegment A und/oder Gehäusesegment C mindestens einen Bereich ohne Beschichtung aus elektrisch isolierendem aufweist. Vorzugsweise grenzt der Bereich dabei nicht an Gehäusesegment B. In einer Ausführung des erfindungsgemäßen elektromedizinischen Implantats weisen Gehäusesegment A und/oder Gehäusesegment C zumindest teilweise eine Beschichtung auf, die die elektrische Kopplung verbessert, wie z. B. eine fraktale Beschichtung mit oberflächenvergrößernden Eigenschaften, und/oder die Beschichtung weist bioaktive Eigenschaften auf.

Vorteilhafte Materialien für eine derartige Beschichtung stellen beispielsweise Silikone, Parylene, Diamond-like Carbon (DLC), Lacke, Pulverbeschichtungen oder Nanobeschichtungen dar, bzw. eine Mischung oder Kombination aus genannten Materialien.

Eine solche Beschichtung kann dazu dienen, den elektrischen Pfad von Gehäusesegment A zu C über das Gewebe (dieser Pfad stellt den elektrischen Mess- und/oder Stimulationsvektor dar) zu bestimmen. Dazu werden die Stellen, an denen der Strom ein- bzw. aus dem Gehäusesegment austreten soll, nicht beschichtet. Vorzugsweise wird der Strompfad für den Mess- und/oder Stimulationsvektor so gewählt, dass er hinreichend lang ist. Wenn das erfindungsgemäße elektromedizinische Implantat eine längliche Form aufweist, so sind die benannten Austrittsstellen vorzugsweise so ausgelegt, dass sie sich an den Enden der Längsachse des Implantats befinden.

Gemäß einer bevorzugten Ausführungsform des erfindungsgemäßen elektromedizinischen Implantats besteht die elektrische Durchführung, bzw. der Durchführungskörper der elektrischen Durchführung aus einem keramischen Material und/oder einem Kompositmaterial. Beispielhafte geeignete Materialien sind Al2O3, ZrO2, Ti2O. Ebenfalls denkbare Materialien für den angegebenen Zweck stellen Materialien der Multilagen-Technologie dar, beispielsweise Keramikmaterialien der Multilagen-Technologie (High Temperature Co-fired Ceramics HTCC, Low Temperature Co-fired Ceramics LTCC).

In einer bevorzugten Ausgestaltung der vorliegenden Erfindung weist das elektromedizinische Implantat mindestens ein aktives oder passives Bauelement auf, wobei das Bauelement in der Durchführung integriert ist und/oder mit der Durchführung verbunden ist.

Beispielhafte passive oder aktive elektronische Bauelemente stellen passive oder aktive Filter dar, sowie passive oder aktive Baugruppen für Hochfrequenz (HF) Anwendungen wie z.B. für Signalübertragung mit externen Geräten oder Signalübertragung innerhalb eines Verbundes aktiver Implantate und/oder passive oder aktive Baugruppen für das Management der Batterie.

Gemäß einer bevorzugten Ausführungsform weist das erfindungsgemäße elektromedizinische Implantat eine Antenne auf, die in der Durchführung integriert ist und/oder mit der Durchführung verbunden ist. Die Antenne dient zum Senden und Empfangen von Daten zu/von einem externen System oder einem externen Gerät zum Zweck der Telemetrie. Dabei sind hier sämtliche, für den Fachmann als zweckdienlich zu erwägende und dabei an oder in die Durchführung integrierbare Antennenarten und - formen denkbar.

In einer Ausführungsform weist das erfindungsgemäße elektromedizinische Implantat eine Antenne auf, welche durch die zwei voneinander elektrisch isolierten Gehäusesegmente A und C des elektromedizinischen Implantats (und der damit entstehende elektrische Dipol) gebildet wird.

In einer vorteilhaften Ausgestaltung des erfindungsgemäßen elektromedizinischen Implantats ist das Gehäusesegment A mit Gehäusesegment B verbunden und/oder Gehäusesegment B ist mit Gehäusesegment C verbunden mittels einer Niedrigtemperatur-Lötung, Laserlötung, Kaltschweißverbindung, Reibschweißverbindung, Schmelzschweißverbindung, Klebung oder Ultraschallverbindung.

Gemäß einer bevorzugten Ausführungsform des erfindungsgemäßen elektromedizinischen Implantats weist die elektrische Durchführung einen Aufbau aus mehreren Schichten auf, wobei die Schichten abwechselnd aus elektrisch isolierendem und elektrisch leitendem Material bestehen. Bei Verwendung einer solchen Durchführung ist in einer weiteren Ausgestaltung denkbar, weitere Elektrodenpole zur Messung und/oder Stimulation an den Außenflächen der Schichten aus elektrisch leitendem Material zu platzieren, sodass multipolare Messungen und/oder Stimulationen durchgeführt werden können. Auch lassen sich passive oder aktive elektronische Bauelemente innerhalb des Aufbaus aus mehreren Schichten implementieren.

Des Weiteren wird ein Verfahren zur Herstellung eines elektromedizinischen Implantats beansprucht, welches zumindest die folgenden Schritte umfasst:
- Bereitstellung eines Elektronikmoduls, umfassend elektrische Schaltung zur Aufnahme und Weiterverarbeitung von elektrischen Signalen mit mindestens zwei elektrischen Leitern zur Messung von elektrischen Potentialen,
- Bereitstellung eines Batteriemoduls, einer elektrischen Durchführung, wobei die Durchführung mindestens teilweise aus elektrisch isolierendem Material besteht, einer Gehäuseschale A und einer Gehäuseschale C, wobei Gehäuseschalen A und C mindestens teilweise aus elektrisch leitendem Material bestehen,
- Anordnen der Durchführung zwischen Elektronikmodul und Batteriemodul,
- Herstellen einer elektrischen Verbindung zwischen Elektronikmodul und Batteriemodul über die Durchführung,
- Herstellen einer elektrischen Verbindung zwischen der elektrischen Schaltung und dem aus elektrisch leitendem Material bestehenden Teil der Gehäuseschale A,
- Herstellen einer elektrischen Verbindung zwischen der elektrischen Schaltung und dem aus elektrisch leitendem Material bestehenden Teil der Gehäuseschale C über die Durchführung,
- Anbinden von Gehäuseschale A an der zum Elektronikmodul gelegenen Seite der Durchführung, sodass das Elektronikmodul innerhalb der Gehäuseschale A angeordnet und hermetisch abgeschlossen ist,
- Anbinden von Gehäuseschale C an der zum Batteriemodul gelegenen Seite der Durchführung, sodass das Batteriemodul innerhalb der Gehäuseschale C angeordnet und hermetisch abgeschlossen ist, sodass
- Gehäuseschale A und Gehäuseschale C durch die Durchführung voneinander elektrisch isoliert sind.

In diesem Zusammenhang soll unter einer "Gehäuseschale" ein geometrischer Hohlkörper mit mindestens einer Öffnung verstanden werden, welcher vorteilhaft als Teil eines Gehäuses für ein Implantat gemäß der vorliegenden Erfindung verwendet werden kann. Als vorteilhaft können Formen der Gehäuseschale angesehen werden, die nach der Implantation für einen hohen Patientenkomfort sorgen sowie Formen, die mit einem geringen Infektionsrisiko nach der Implantation einhergehen und/oder solche, die einfach zu implantieren sind und nach der Implantation fest an ihrem Implantationsort verbleiben, ohne dass sie verrutschen oder sich verdrehen. Dabei bietet es sich an, wenn die Form der Gehäuseschale eine kleine Gesamtaußenfläche relativ zum Innenvolumen aufweist. Auch bieten sich Formen mit wenigen Ecken und Kanten an, bzw. mit abgerundeten Ecken und Kanten. Beispielsweise kann eine Gehäuseschale als hohler Quader mit einer offenen Seitenwand oder Paraboloid ausgeführt sein. Dabei können die Gehäuseschalen A und/oder C aus einem einzigen Material bestehen, wie zum Beispiel aus Metall oder einem anderen dem Fachmann als zweckdienlich erscheinenden, elektrisch leitenden Material, welches vorzugsweise Biokompatibilität aufweist. Des Weiteren ist auch denkbar, dass eine Gehäuseschale verschiedene Materialien umfasst, wie beispielsweise aus einem Materialverbund besteht. Mögliche Materialien geeignete Materialien sind zum Beispiel Metall-Kunststoff Verbunde, Metall- Keramik Verbunde, Metall-Glas Verbunde. Die elektrische Durchführung besitzt einen Durchführungskörper, der vorzugsweise zumindest teilweise aus elektrisch isolierendem Material besteht. Geeignet für diesen Zweck wären beispielsweise Glas, Keramik, Kunststoffe bzw. Verbundmaterialien aus den genannten.

Das Elektronikmodul im Sinne der des erfindungsgemäßen Verfahrens ist im Inneren der Gehäuseschale A angeordnet und umfasst eine elektrische Schaltung. Diese beinhaltet beispielsweise elektrische Schaltkreise für die Aufnahme und Verarbeitung von elektrischen Potentialen sowie Einheiten zur Speicherung, Signalweiterverarbeitung und Signalanalyse. Des Weiteren kann der Schaltkreis Komponenten für die Abgabe von elektrischen Pulsen sowie zur Koordination dieser Pulse beinhalten. Ebenfalls denkbar wären Schaltkreiskomponenten für die drahtlose Datenübertragung (Telemetrie) von gemessenen und gespeicherten Daten an externe Systeme oder ein externes Patientengerät. Weiterhin umfasst das Elektronikmodul mindestens einen ersten und zweiten Leiter. Die Leiter sind mit ihrem jeweiligen Leiteranfang mit der elektrischen Schaltung verbunden.

Im Zusammenhang des erfindungsgemäßen Verfahrens soll unter dem "Herstellen einer elektrischen Verbindung" allgemein die Implementierung einer elektrischen Leiterverbindung verstanden werden, sodass elektronische Elemente kontaktiert werden. Eine solche Leiterverbindung kann beispielsweise als Leiterdraht mit Leiteranfang und Leiterende aufgeführt sein, oder als Leiterbahn auf einer Platine mit elektrischer Kontaktierungsmöglichkeit am Anfang und Ende der Leiterbahn, oder als leitender Teil einer elektrischen Durchführung ausgelegt sein, oder einer Kombination aus den genannten und weiteren, für den Fachmann zweckdienlichen Möglichkeiten.

Nach dem erfindungsgemäßen Verfahren ist das Batteriemodul im Inneren der Gehäuseschale C angeordnet. In einer bevorzugten Ausführungsform besteht keine direkte elektrische Verbindung zwischen dem Batteriemodul und Gehäuseschale C.

Im Zusammenhang mit dem erfindungsgemäßen Verfahren wird eine elektrische Durchführung für das elektromedizinische Implantat bereitgestellt. Im Sinne der Erfindung wird unter einer elektrischen Durchführung ein Element zur elektrischen Leitung verstanden, welches ausgelegt ist, ein elektrisches Signal von einer Umgebung in eine andere überzuführen bzw. einzukoppeln, wobei Signaleigenschaften und -Qualität so gut wie möglich erhalten bleiben sollen. Beispielsweise kann es sich dabei um die Einkopplung eines Signals in ein Gerätegehäuse, oder von einem Gehäuseabschnitt in einen anderen Gehäuseabschnitt handeln. Im Sinne der Erfindung besteht die elektrische Durchführung aus einem Durchführungskörper, in den elektrische Leiterelemente integriert sind bzw. durch den elektrische Leiterelemente durchführen. Dabei führen die Leiterelemente jeweils von einer ersten Außenfläche des Durchführungskörpers durch den Durchführungskörper hindurch zu einer zweiten Außenfläche des Durchführungskörpers. Die erste und die zweite Außenfläche können hierbei dieselbe Außenfläche oder unterschiedliche Außenflächen des Durchführungskörpers darstellen. Die Leiterelemente verfügen an ihren Enden über elektrische Kontakte, die an der entsprechenden Außenfläche überstehen und über diese das elektrische Signal in die Durchführung eingekoppelt werden kann. In einer Ausführungsform werden besagte elektrische Kontakte durch Anlöten am Durchführungskörper angebunden werden. Bevorzugt besteht der Durchführungskörper mindestens teilweise aus einem elektrisch isolierenden Material. In einer Ausführungsform besteht der gesamte Durchführungskörper aus einem solchen Material. Auf jeden Fall jedoch ist im Sinne des erfindungsgemäßen Verfahrens die elektrische Durchführung bzw. der Durchführungskörper ausgelegt, Gehäuseschalen A und C elektrisch voneinander zu isolieren, sodass kein direkter Strompfad von Gehäuseschale A über die Durchführung zur Gehäuseschale C besteht. Innerhalb des Gehäuses ist, wie zuvor erwähnt, das Elektronikmodul im Inneren der Gehäuseschale A angeordnet, während das Batteriemodul im Inneren der Gehäuseschale C angeordnet ist. Dabei sind das Elektronikmodul und das Batteriemodul vorzugsweise elektrisch über/durch die Durchführung miteinander verbunden. Dies kann durch zusätzliche elektrische Leiter gewährleistet werden, die das Elektronikmodul mit elektrischen Kontakten an der zum Elektronikmodul gerichteten Seite der Durchführung verbinden, sowie zusätzliche elektrische Leiter, die das Batteriemodul mit elektrischen Kontakten an der zum Batteriemodul gerichteten Seite der Durchführung verbinden.

Im Sinne des erfindungsgemäßen Verfahrens wird die Durchführung zwischen Elektronikmodul und Batteriemodul angeordnet. Das Elektronikmodul wird mit dem Batteriemodul über die elektrischen Leitungen in der Durchführung miteinander elektrisch verbunden, weiterhin werden elektrische Verbindungen hergestellt zwischen der elektrischen Schaltung und dem aus elektrisch leitendem Material bestehenden Teil der Gehäuseschale A sowie zwischen der elektrischen Schaltung und dem aus elektrisch leitendem Material bestehenden Teil der Gehäuseschale C über die Durchführung. Gehäuseschale A und C werden jeweils an der zum Elektronikmodul bzw. zum Batteriemodul gelegenen Seite der Durchführung angebunden. Im Sinne der Erfindung wird unter "Anbinden" vorzugsweise ein Zusammenfügen der entsprechenden Komponenten verstanden, sodass ein stabiler, fester Materialverbund entsteht, wie beispielsweise durch Löten, Schweißen oder Kleben. Gemäß dem vorgeschlagenen Verfahren sind Gehäuseschale A und C somit durch die Durchführung elektrisch voneinander isoliert. Gemäß dem vorschlagsgemäßen Verfahren wird zwischen Gehäuseschale A bzw. C und dem Elektronikmodul bzw. dem Batteriemodul ein Strompfad erzeugt, der von der elektrischen Schaltung des Elektronikmoduls zum elektrisch leitenden Teil der Gehäuseschale A führt, von dort aus zum elektrisch leitenden Teil der Gehäuseschale C, dann durch die Durchführung zurück zur elektrischen Schaltung. Folglich werden die zwei für die Messung bzw. Abgabe von elektrischen Pulsen erforderlichen Elektrodenpole gebildet durch den elektrisch leitenden Teil von Gehäuseschale A und Gehäuseschale C. Wie zuvor erwähnt können dabei Gehäuseschale A und/oder C auch gänzlich aus elektrisch leitendem Material bestehen.

In einer vorteilhaften Ausführung des erfindungsgemäßen Verfahrens umfasst das Verfahren die Kontaktierung der Gehäuseschalen A und C mittels weiterer elektrischer Leiter, welche durch die Durchführung führen. Dabei ist in einer Ausführungsform denkbar, dass Gehäuseschalen A und C (oder der elektrisch leitende Teil der jeweiligen Gehäuseschale) jeweils fest auf dem Plus- bzw. Minuspol der Batterie des Batteriemoduls liegen. Auch denkbar ist es, dass das elektrische Potential der elektrisch leitenden Teile der Gehäuseschalen A und C veränderlich (auf Englisch ,floating') gestaltet sind.

In einer bevorzugten Ausführungsform des erfindungsgemäßen Verfahrens ist das elektromedizinische Implantat als kardiales Implantat in Form eines Sensors, Überwachungsgeräts, Schrittmachergeräts, Schrittmachergeräts mit integrierter Defibrillatorfunktion oder Schrittmachergeräts ohne intrakardiale Elektroden ausgeführt.

Gemäß einer vorteilhaften Ausgestaltung umfasst das erfindungsgemäße Verfahren zur Herstellung eines elektromedizinischen Implantats zumindest den weiteren Schritt der Beschichtung der Gehäuseschale A und/oder der Gehäuseschale C mit elektrisch isolierendem Material, wobei ein Bereich der Gehäuseschale A und/oder Gehäuseschale C nicht beschichtet wird, wobei der Bereich nicht an die Durchführung angrenzt.

Vorteilhafte Materialien für eine derartige Beschichtung stellen beispielsweise Silikone, Parylene, Diamond-like Carbon (DLC), Lacke, Pulverbeschichtungen oder Nanobeschichtungen dar, bzw. eine Mischung oder Kombination aus genannten Materialien.

Eine solche Beschichtung kann dazu dienen, den elektrischen Pfad von Gehäuseschale A zu C über das Gewebe (dieser Pfad stellt den elektrischen Mess- und/oder Stimulationsvektor dar) zu bestimmen. Dazu werden die Stellen, an denen der Strom ein- bzw. aus der Gehäuseschale austreten soll, nicht beschichtet. Vorzugsweise wird der Strompfad für den Mess- und/oder Stimulationsvektor so gewählt, dass er hinreichend lang ist. Wenn das erfindungsgemäße elektromedizinische Implantat eine längliche Form aufweist, so sind die benannten Austrittsstellen vorzugsweise so ausgelegt, dass sie sich an den Enden der Längsachse des Implantats befinden.

In einer bevorzugten Ausführungsform für das erfindungsgemäße Verfahren zur Herstellung eines elektromedizinischen Implantats erfolgt das Anbinden der Gehäuseschale A und/oder Gehäuseschale C durch eine Niedrigtemperatur-Lötung, Laserlötung, Kaltschweißen, Reibschweißen, Schmelzschweißen, Klebung oder Ultraschallschweißen.

Gemäß einer vorteilhaften Ausgestaltung umfasst das erfindungsgemäße Verfahren zur Herstellung eines elektromedizinischen Implantats zumindest den weiteren Schritt der Integration von mindestens einem passiven oder aktiven Bauelement und/oder einer Antenne in der Durchführung. Die Antenne dient zum Senden und Empfangen von Daten zu/von einem externen System oder einem externen Gerät zum Zweck der Telemetrie. Dabei sind hier sämtliche, für den Fachmann als zweckdienlich zu erwägende und dabei an oder in die Durchführung integrierbare Antennenarten und -formen denkbar.

In einer bevorzugten Ausführungsform des erfindungsgemäßen Verfahrens weist die elektrische Durchführung einen Aufbau aus mehreren Schichten auf, wobei die Schichten abwechselnd aus elektrisch isolierendem und elektrisch leitendem Material bestehen. Bei Verwendung einer solchen Durchführung ist in einer weiteren Ausgestaltung denkbar, weitere Elektrodenpole zur Messung und/oder Stimulation an den Außenflächen der Schichten aus elektrisch leitendem Material zu platzieren, sodass multipolare Messungen und/oder Stimulationen durchgeführt werden können. Auch lassen sich passive oder aktive elektronische Bauelemente innerhalb des Aufbaus aus mehreren Schichten implementieren.

In einer bevorzugten Ausgestaltung des erfindungsgemäßen Verfahrens zur Herstellung eines elektromedizinischen Implantats ist Gehäuseschale A und/oder Gehäuseschale C aus zwei zusammengefügten Halbschalen aufgebaut. Ein solcher modularer Aufbau der Gehäuseschalen ermöglicht eine vereinfachte Anfertigung der Gehäuseschalen. Beispielsweise können die Halbschalen zu einer Gehäuseschale durch Schweißen zusammengefügt werden.

Die Erfindung ist nachfolgend beispielhaft, anhand von einem in Zeichnungen dargestellten Ausführungsbeispiel, näher erläutert. Es zeigen in schematischer Darstellung:
- Fig. 1a: Äußerer Aufbau eines beispielhaften implantierbaren Geräts zur Messung von elektrischen Potentialen aus dem Stand der Technik;
- Fig. 1b: Äußerer und innerer Aufbau eines beispielhaften implantierbaren Geräts zur Messung von elektrischen Potentialen aus dem Stand der Technik;
- Fig. 2a: Äußerer Aufbau eines beispielhaften elektromedizinischen Implantats gemäß der Erfindung;
- Fig. 2b: Äußerer und innerer Aufbau eines beispielhaften elektromedizinischen Implantats gemäß der Erfindung;
- Fig. 3: Bevorzugte Anbindung zwischen der elektrischen Durchführung und den angrenzenden Gehäusesegmenten gemäß der Erfindung;
- Fig. 4: Vorteilhafte Ausführungsform der elektrischen Verbindung und Verschaltung für das elektromedizinische Implantat gemäß der Erfindung;
- Fig. 5: Beispielhafte Ausführungsformen für die elektrische Durchführung des elektromedizinischen Implantats gemäß der Erfindung;
- Fig. 6: Beispielhafte Ausführungsform für die elektrische Durchführung des elektromedizinischen Implantats mit mehrschichtigem Aufbau gemäß der Erfindung;
- Fig. 7: Ausführungsform der Gehäusesegmente als Gehäuseschalen, die Halbschalen zusammengesetzt sind.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

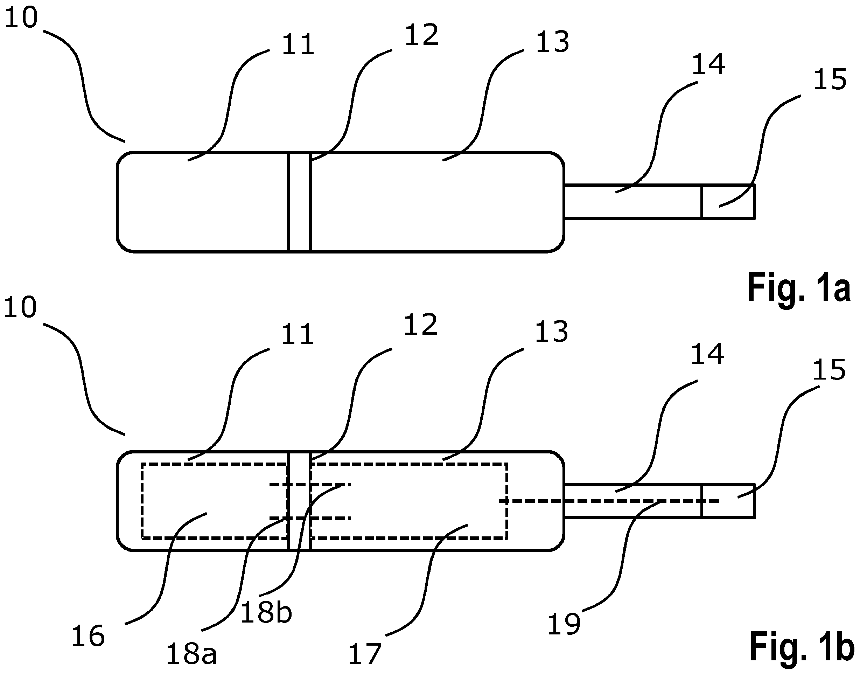

Fig. 1a und 1b zeigen ein beispielhaftes implantierbares Gerät 10 zur Messung von elektrischen Potentialen aus dem Stand der Technik. Dabei wird in Fig. 1a der äußere Aufbau eines solchen implantierbaren Geräts 10 illustriert, mit einem Gehäuse bestehend aus Batteriegehäuse 11, einem Batteriedeckel mit ersten Durchführung 12 und Elektronikgehäuse 13. Des Weiteren weist das implantierbare Gerät 10 eine zweite Durchführung 14 und einer Messelektrode 15 auf. In Fig. 1b wird gestrichelt der Innenaufbau des implantierbaren Geräts 10 gezeigt, bestehend aus Batteriemodul 16, Elektronikmodul 17, elektrischen Verbindungen 18a und 18b, welche Batteriemodul 16 und Elektronikmodul 17 über die Durchführung im Batteriedeckel 12 elektrisch verbinden, sowie elektrische Verbindung 19, welche das Elektronikmodul mit der Messelektrode 15 verbindet. Die Gegenelektrode zur Messelektrode 15 kann dabei vom Gehäuse gebildet werden.

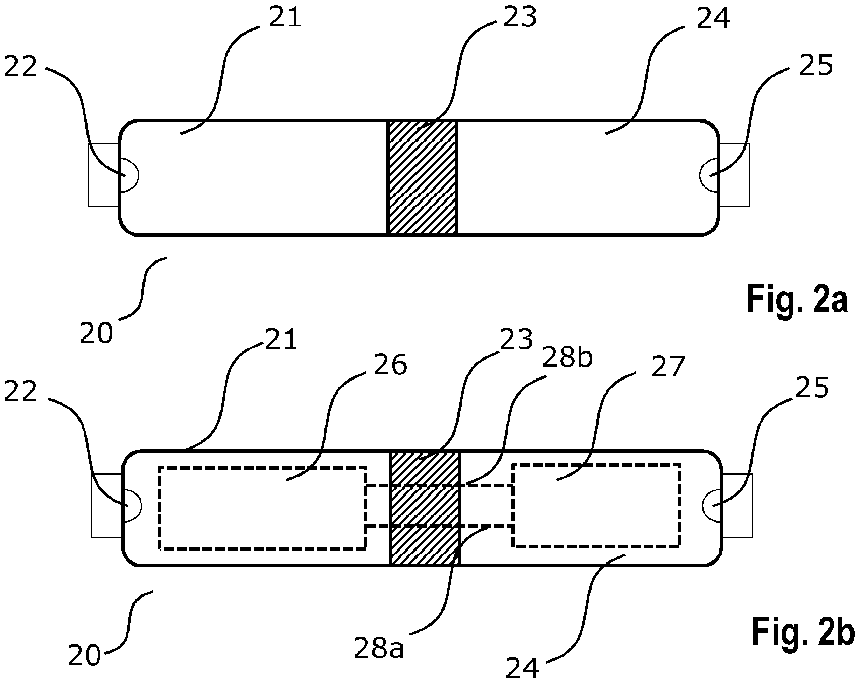

Fig. 2a und 2b zeigen ein Ausführungsbeispiel eines elektromedizinischen Implantats 20 im Sinne der Erfindung. Dabei illustriert Fig. 2a den äußeren Aufbau des elektromedizinischen Implantats 20, bestehend aus einem ersten Gehäusesegment 21 mit einem aus elektrisch leitendem Material bestehenden Teil 22, einer elektrischen Durchführung 23, einem zweiten Gehäusesegment 24 mit einem aus elektrisch leitendem Material bestehenden Teil 25. Fig. 2b zeigt schematisch den Innenaufbau (gestrichelt) des Ausführungsbeispiels in Fig. 2a, umfassend ein Batteriemodul 26, ein Elektronikmodul 27 sowie zwei elektrische Verbindungen 28a und 28b, die Batteriemodul 26 und Elektronikmodul 27 über die Durchführung 23 elektrisch verbinden. Weitere für die Funktionalität der Erfindung vorgesehene elektrische Verbindungen und Verschaltungen sind in Fig. 2b aus Übersichtsgründen nicht eingezeichnet. Die aus elektrisch leitendem Material bestehenden Teile 22 und 25 der Gehäusesegmente 21 und 24 stellen die elektrischen Pole für die Messung bzw. die Stimulation dar. Beim beispielhaften elektromedizinischen Implantat 20 befinden sich 22 und 25 an den Enden der Längsachse des elektromedizinischen Implantats. Dies bringt den Vorteil eines verhältnismäßig langen Mess- bzw. Stimulationsvektors, um Messungen mit hohem Informationsgehalt durchführen zu können, d. h. um repräsentativere Messungen zu erhalten. Alternativ können Gehäusesegmente 21 und 24 auch komplett aus elektrisch leitendem Material bestehen. 22 und 25 können dann implementiert werden, indem die Gehäusesegmente 21 und 24 mit einer elektrisch isolierenden Beschichtung versehen werden, wobei die Stellen an 22 und 25 nicht beschichtet werden. Im Ausführungsbeispiel des elektromedizinischen Implantats 20 sind die Gehäusesegmente 21 und 24 als längliche Halbschalen ausgeführt, die an ihrem offenen Ende mit der Durchführung verbunden sind. In diesem Beispiel ist das Innere des elektromedizinischen Implantats 20 durch Gehäusesegmente 21 und 24 in Verbindung mit der Durchführung 23 hermetisch abgeschlossen.

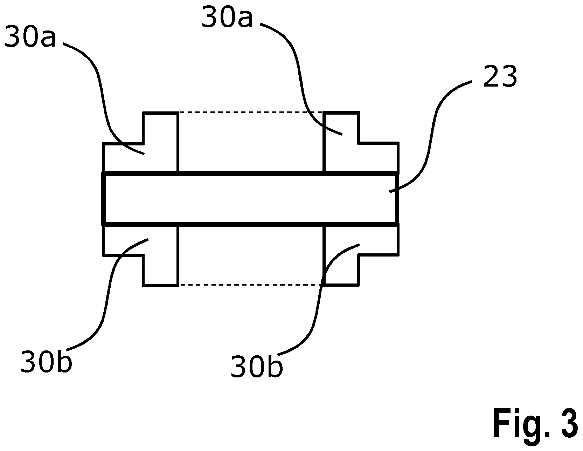

Fig. 3 zeigt eine bevorzugte Ausführungsform der Anbindung zwischen Durchführung 23 mit den angrenzenden Gehäusesegmenten 21 und 24 in der Schnittansicht. Zum Beispiel können auf der Durchführung 23 zunächst metallische Anschluss-Kontakte angelötet werden, um dann die Gehäusesegmente an den entsprechenden Schnittstellen 30a und 30b an die Durchführung anzubinden, beispielsweise mittels Laserschweißen.

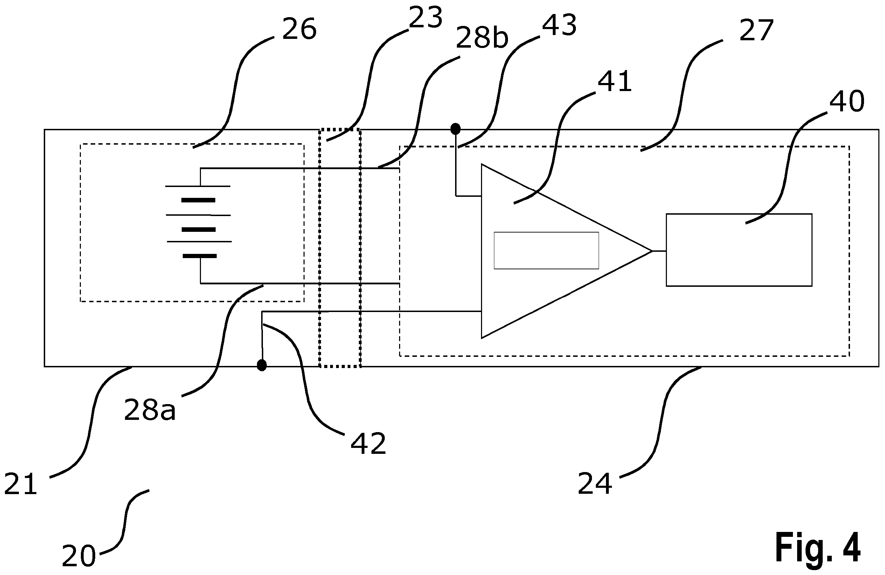

Fig. 4 zeigt eine vorteilhafte Ausführungsform der elektrischen Verbindung und Verschaltung für das elektromedizinische Implantat gemäß der Erfindung. Das elektromedizinische Implantat 20 weist, entsprechend der Ausführung in Fig. 2a und 2b, ein erstes Gehäusesegment 21, eine elektrische Durchführung 23 und ein zweites Gehäusesegment 24 auf. Im Inneren des elektromedizinischen Implantats 20 befinden sich Batteriemodul 26, ein Elektronikmodul 27 sowie zwei elektrische Verbindungen 28a und 28b, die Batteriemodul 26 und Elektronikmodul 27 über die Durchführung 23 elektrisch verbinden. Das Elektronikmodul verfügt über eine elektrische Schaltung 40, die beispielsweise elektrische Schaltkreise für die Aufnahme und Verarbeitung von elektrischen Potentialen sowie Einheiten zur Speicherung, Signalweiterverarbeitung und Signalanalyse beinhalten kann. Des Weiteren kann der Schaltkreis Komponenten für die Abgabe von elektrischen Pulsen sowie zur Koordination dieser Pulse beinhalten. Ebenfalls denkbar wären Schaltkreiskomponenten für die drahtlose Datenübertragung (Telemetrie) von gemessenen und gespeicherten Daten an externe Systeme oder ein externes Patientengerät. Weiterhin umfasst das Elektronikmodul 27 mindestens einen ersten Leiter 42 und einen zweiten Leiter 43. Leiter 42 ist elektrisch mit Gehäusesegment 21 bzw. mit dem aus elektrisch leitendem Material bestehenden Teil des Gehäusesegments 21 verbunden, führt durch die elektrische Durchführung 23 zum Elektronikmodul 27. Dabei kann Leiter 42 in den ersten Eingang eines Operationsverstärkers 41 führen. Leiter 43 ist elektrisch mit Gehäusesegment 24 bzw. mit dem aus elektrisch leitendem Material bestehenden Teil des Gehäusesegments 24 verbunden und führt zum Elektronikmodul 27. Dabei kann Leiter 41 in einen zweiten Eingang des Operationsverstärkers 41 führen. Die aufgenommenen elektrischen Potentiale über Leiter 42 und 43 werden von Verstärker 41 verstärkt und zur elektrischen Schaltung 40 weitergeleitet, wo sie weiterverarbeitet werden.

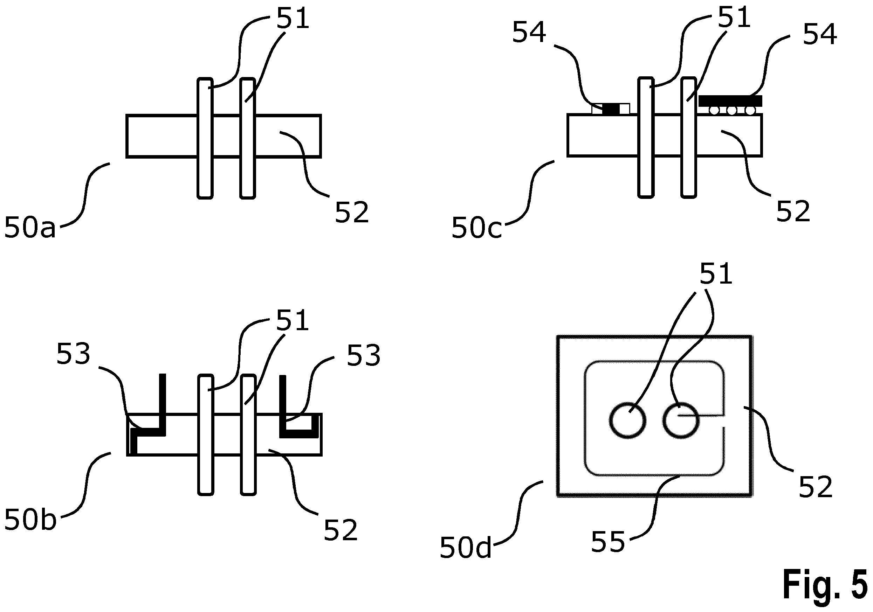

Fig. 5 zeigt schematisch beispielhafte Ausführungsformen 50a, 50b und 50c für die elektrische Durchführung des elektromedizinischen Implantats gemäß der Erfindung in der Schnittansicht. Ausführungsform 50d zeigt die elektrische Durchführung des elektromedizinischen Implantats in der Draufsicht. Durchführungsvariante 50a besteht aus einem Durchführungskörper 52 und zwei elektrischen Kontakten 51, die durch den Durchführungskörper führen und an dessen Enden elektrische Kontakte angebracht werden können (zum Beispiel durch Anlöten). Durchführungsvariante 50b umfasst zusätzliche elektrische Bahnen 53, welche durch den Durchführungskörper 52 hindurchführen. Diese zusätzlichen Leiter können für weitere Modifikationen und Anpassungen der elektrischen Potentiale der Gehäusesegmente verwendet werden, beispielsweise um die elektrischen Potentiale dynamisch veränderlich zu gestalten (zu Englisch ,floating'). Durchführungsvariante 50c umfasst weitere Bauelemente 54, die am oder im Durchführungskörper 52 integriert werden können und/oder mit dem Durchführungskörper 52 verbunden sein können. Beispielhafte Bauelemente sind elektronische passive oder aktive Bauelemente wie [s.o.], oder Antennenformen für den Zweck der Telemetrie. Ausführungsbeispiel 50d zeigt eine Variante der Durchführung, bei der eine Antenne 55 in der Durchführung integriert ist, so kann beispielsweise Antenne 55 in den Durchführungskörper 52 eingearbeitet sein, oder Antenne 55 ist mit der Durchführung verbunden, beispielsweise auf die Oberfläche des Durchführungskörpers 52 aufgebracht.

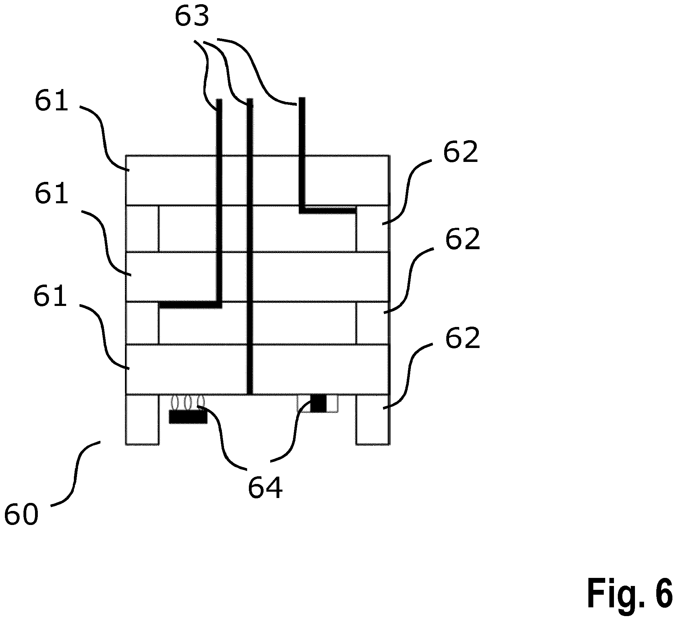

Fig. 6 zeigt schematisch eine beispielhafte Ausführungsform 60 für die elektrische Durchführung des elektromedizinischen Implantats mit mehrschichtigem Aufbau gemäß der Erfindung in der Schnittansicht. Die Durchführung 60 besteht aus mehreren alternierenden Schichten aus elektrisch isolierendem Material 61 und elektrisch leitendem Material 62. Elektrische Leiter 63 führen durch die Schichten und können so kontaktiert werden, dass weitere elektrische Pole an den Schichten aus elektrisch leitendem Material 62 entstehen können. Über diese Pole können weitere elektrische Potentiale gemessen bzw. elektrische Pulse abgegeben werden. Des Weiteren kann auch Durchführung 60 weitere elektronische Bauelemente 64 umfassen, die an und/oder in den Schichten integriert und/oder mit den Schichten verbunden sind. Beispielhafte Bauelemente sind elektronische passive oder aktive Bauelemente oder Antennenformen für den Zweck der Telemetrie.

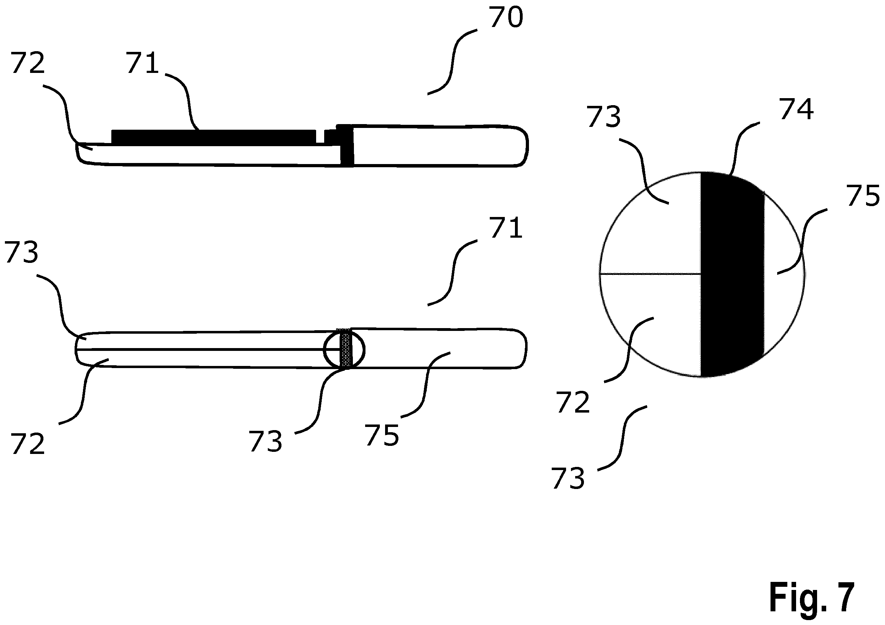

Fig. 7 zeigt eine Ausführungsform des elektromedizinischen Implantats im Sinne der Erfindung, bei welcher die Gehäusesegmente als Gehäuseschalen ausgeführt sind, wobei eine Gehäuseschale aus zwei Halbschalen zusammengesetzt ist. 70 und 71 zeigen Seitenansichten der Ausführungsform des elektromedizinischen Implantats, wobei das Gehäusesegment, welches das Modul 71 beherbergt, aus zwei Halbschalen 72 und 73 zusammengefügt ist. Das Modul 71 kann dabei das Batteriemodul oder das Elektronikmodul gemäß der Erfindung darstellen. Bereich 73 zeigt die Stelle, an der die Halbschalen 72 und 73 an Durchführung 74 grenzen, im Detail. Gehäusesegment 75 ist eine Ausführungsform des erfindungsgemäßen Gehäusesegments als Gehäuseschale und kann auch aus zwei Halbschalen bestehen. Denkbar wäre auch ein Aufbau eines Gehäusesegments gemäß der Erfindung aus mehr als zwei Komponenten. Das Zusammenfügen der Halbschalen 72 und 73, bzw. das Anfügen der Halbschalen 72 und 73 an die Durchführung 74 kann durch Fügeverfahren wie beispielsweise einer Niedrigtemperatur-Lötung, Laserlötung, Kaltschweißen, Reibschweißen, Schmelzschweißen, Klebung oder Ultraschallschweißen umgesetzt werden.

### Bezugszeichen

- 10: implantierbares Gerät aus dem Stand der Technik
- 11: Batteriegehäuse
- 12: erste Durchführung
- 13: Elektronikgehäuse
- 14: zweite Durchführung
- 15: Messelektrode
- 16: Batteriemodul
- 17: Elektronikmodul
- 18a, 18b: elektrische Verbindungen
- 19: elektrische Verbindung
- 20: elektromedizinisches Implantat
- 21: erstes Gehäusesegment
- 22: aus elektrisch leitendem Material bestehender Teil des Gehäusesegments 21
- 23: elektrische Durchführung
- 24: zweites Gehäusesegment
- 25: aus elektrisch leitendem Material bestehender Teil des Gehäusesegments 24
- 26: Batteriemodul
- 27: Elektronikmodul
- 28a, 28b: elektrische Verbindungen
- 30a, 30b: Schnittstellen der Gehäusesegmente
- 40: elektrische Schaltung
- 41: Verstärker
- 42: erster Leiter
- 43: zweiter Leiter
- 50a, 50b, 50c: beispielhafte Ausführungsformen für elektrische Durchführung
- 51: elektrische Kontakte
- 52: Durchführungskörper
- 53: elektrische Bahnen
- 54: Bauelemente
- 60: beispielhafte Ausführungsform der elektrischen Durchführung
- 61: elektrisch isolierendes Material
- 62: elektrisch leitendes Material
- 63: elektrische Leiter
- 64: elektronische Bauelemente
- 70: Ausführungsform des elektromedizinischen Implantats in Seitenansicht
- 71: Ausführungsform des elektromedizinischen Implantats in Seitenansicht
- 72: Halbschale
- 73: Halbschale
- 74: Durchführung
- 75: Gehäusesegment

## Patentansprüche

1. Elektromedizinisches Implantat (20), umfassend
ein Gehäuse, aufweisend
drei Gehäusesegmente A (21), B und C (24), wobei
Gehäusesegment A (21) verbunden ist mit Gehäusesegment B, und Gehäusesegment B verbunden ist mit Gehäusesegment C (24), sodass Gehäusesegment B zwischen den Gehäusesegmenten A (21) und C (24) angeordnet ist, wobei Gehäusesegment A (21) sowie Gehäusesegment C (24) mindestens teilweise aus elektrisch leitendem Material (22, 25) bestehen,
ein Elektronikmodul (27), das innerhalb des Gehäusesegments A (21) angeordnet ist, umfassend
eine elektrische Schaltung (40) zur Aufnahme und Weiterverarbeitung von elektrischen Signalen und
mindestens ein erster und ein zweiter elektrischer Leiter (42, 43) zur Messung von elektrischen Potentialen und/oder Abgabe von elektrischen Pulsen, wobei jeder Leiter jeweils einen Leiteranfang und ein Leiterende aufweist und jeder Leiteranfang mit der elektrischen Schaltung (40) verbunden ist,
ein Batteriemodul (26), wobei das Batteriemodul ein Batteriegehäuse aufweist, welches durch Gehäusesegment C (24) gebildet wird,
wobei das elektromedizinische Implantat (20) eine elektrische Durchführung (23) aufweist, wobei die elektrische Durchführung (23) mindestens teilweise aus elektrisch isolierendem Material besteht und Gehäusesegment B durch Teile der Durchführung (23) gebildet wird,
wobei Elektronikmodul (27) und Batteriemodul (26) über die Durchführung (23) elektrisch verbunden sind, und
Gehäusesegment A (21) und Gehäusesegment C (24) durch die Durchführung (23) voneinander elektrisch isoliert sind,
und wobei das Leiterende des ersten elektrischen Leiters (42) über die Durchführung (23) mit dem aus elektrisch leitendem Material bestehenden Teil (25) des Gehäusesegments C elektrisch verbunden ist,
**dadurch gekennzeichnet, dass**
das Leiterende des zweiten elektrischen Leiters (43) mit dem aus elektrisch leitendem Material bestehenden Teil (22) des Gehäusesegments A elektrisch verbunden ist.

2. Elektromedizinisches Implantat nach Anspruch 1, wobei das elektromedizinische Implantat (20) als Neurostimulationsimplantat in Form eines Rückenmarkstimulationsgeräts, Vagusnerv-Stimulationsgeräts, oder Hirnstimulationsgeräts ausgeführt ist, oder als Muskelstimulationgerät ausgeführt ist, oder als kardiales Implantat in Form eines Sensors, Überwachungsgeräts, Schrittmachergeräts, Schrittmachergeräts mit integrierter Defibrillatorfunktion oder Schrittmachergeräts ohne intrakardiale Elektroden ausgeführt ist.

3. Elektromedizinisches Implantat nach Anspruch 1 oder 2, wobei Gehäusesegment A (21) und/oder Gehäusesegment C (24) eine Beschichtung aus elektrisch isolierendem Material aufweist, wobei Gehäusesegment A (21) und/oder Gehäusesegment C (24) mindestens einen Bereich ohne Beschichtung aus elektrisch isolierendem Material aufweist.

4. Elektromedizinisches Implantat nach mindestens einem der vorhergehenden Ansprüche, wobei die elektrische Durchführung (23) aus einem Material der Multilagen-Technologie besteht.

5. Elektromedizinisches Implantat nach mindestens einem der vorhergehenden Ansprüche, wobei das elektromedizinische Implantat (20) mindestens ein aktives oder passives Bauelement (54) aufweist, das in der Durchführung (23) integriert ist und/oder mit der Durchführung (23) verbunden ist.

6. Elektromedizinisches Implantat nach mindestens einem der vorhergehenden Ansprüche, wobei das elektromedizinische Implantat (20) eine Antenne aufweist, die in der Durchführung (23) integriert ist und/oder mit der Durchführung (23) verbunden ist.

7. Elektromedizinisches Implantat nach mindestens einem der vorhergehenden Ansprüche, wobei Gehäusesegment A (21) mit Gehäusesegment B und/oder Gehäusesegment B mit Gehäusesegment C (24) verbunden ist mittels einer Niedrigtemperatur-Lötung, Laserlötung, Kaltschweißverbindung, Reibschweißverbindung, Schmelzschweißverbindung, Ultraschallschweißverbindung oder Klebeverbindung.

8. Elektromedizinisches Implantat nach mindestens einem der vorhergehenden Ansprüche, wobei die elektrische Durchführung (23) einen Aufbau aus mehreren Schichten aufweist (60), wobei die Schichten abwechselnd aus elektrisch isolierendem (61) und elektrisch leitendem Material (62) bestehen.

9. Verfahren zur Herstellung eines elektromedizinischen Implantats (20), umfassend die Schritte
- Bereitstellung eines Elektronikmoduls (27), umfassend elektrische Schaltung (40) zur Aufnahme und Weiterverarbeitung von elektrischen Signalen mit mindestens zwei elektrischen Leitern (42, 43) zur Messung von elektrischen Potentialen,
- Bereitstellung eines Batteriemoduls (26), einer elektrischen Durchführung (23), wobei die Durchführung (23) mindestens teilweise aus elektrisch isolierendem Material besteht, einer Gehäuseschale A (21) und einer Gehäuseschale C (24), wobei Gehäuseschale A (21) und C (24) mindestens teilweise aus elektrisch leitendem Material (22, 25) bestehen,
- Anordnen der Durchführung (23) zwischen Elektronikmodul (27) und Batteriemodul (26),
- Herstellen einer elektrischen Verbindung (28a, 28b) zwischen Elektronikmodul (27) und Batteriemodul (26) über die Durchführung (23),
- Herstellen einer elektrischen Verbindung zwischen der elektrischen Schaltung (40) und dem aus elektrisch leitendem Material bestehenden Teil (22) der Gehäuseschale A (21),
- Herstellen einer elektrischen Verbindung zwischen der elektrischen Schaltung (40) und dem aus elektrisch leitendem Material bestehenden Teil (25) der Gehäuseschale C (24) über die Durchführung (23),
- Anbinden von Gehäuseschale A (21) an der zum Elektronikmodul (27) gelegenen Seite der Durchführung (23), sodass das Elektronikmodul (27) innerhalb der Gehäuseschale A (21) angeordnet und hermetisch abgeschlossen ist, und
- Anbinden von Gehäuseschale C (24) an der zum Batteriemodul (26) gelegenen Seite der Durchführung (23), sodass das Batteriemodul (26) innerhalb der Gehäuseschale C (24) angeordnet und hermetisch abgeschlossen ist, sodass
- Gehäuseschale A (21) und Gehäuseschale C (24) durch die Durchführung (23) voneinander elektrisch isoliert sind.

10. Verfahren zur Herstellung eines elektromedizinischen Implantats (20) nach Anspruch 9, wobei das elektromedizinische Implantat (20) als kardiales Implantat in Form eines Sensors, Überwachungsgeräts, Schrittmachergeräts, Schrittmachergeräts mit integrierter Defibrillatorfunktion oder Schrittmachergeräts ohne intrakardiale Elektroden ausgeführt ist.

11. Verfahren zur Herstellung eines elektromedizinischen Implantats (20) nach Anspruch 9 oder 10, umfassend den weiteren Schritt
- Beschichtung der Gehäuseschale A (21) und/oder der Gehäuseschale C (24) mit elektrisch isolierendem Material, wobei ein Bereich der Gehäuseschale A (21) und/oder Gehäuseschale C (24) nicht beschichtet wird.

12. Verfahren zur Herstellung eines elektromedizinischen Implantats (20) nach mindestens einem der Ansprüche 9 bis 11, wobei das Anbinden der Gehäuseschale A (21) und/oder Gehäuseschale C (24) durch eine Niedrigtemperatur-Lötung, Laserlötung, Kaltschweißen, Reibschweißen, Schmelzschweißen, Ultraschallschweißen oder Klebung erfolgt.

13. Verfahren zur Herstellung eines elektromedizinischen Implantats (20) nach mindestens einem der Ansprüche 9 bis 12, umfassend den weiteren Schritt.
- Integration von mindestens einem passiven oder aktiven Bauelement (54) und/oder einer Antenne in der Durchführung (23).

14. Verfahren zur Herstellung eines elektromedizinischen Implantats (20) nach mindestens einem der Ansprüche 9 bis 13, wobei die elektrische Durchführung (23) einen Aufbau aus mehreren Schichten aufweist, wobei die Schichten abwechselnd aus elektrisch isolierendem (61) und elektrisch leitendem Material (62) bestehen.

15. Verfahren zur Herstellung eines elektromedizinischen Implantats (20) nach mindestens einem der Ansprüche 9 bis 14, wobei Gehäuseschale A (21) und/oder Gehäuseschale C (24) aus zwei zusammengefügten Halbschalen (72, 73) aufgebaut ist.

## Claims

1. An electromedical implant (20), comprising:
a housing, comprising:
three housing segments A (21), B and C (24),
housing segment A (21) being joined to housing segment B, and housing segment B being joined to housing segment C (24), so that housing segment B is arranged between housing segments A (21) and C (24), and housing segment A (21) and housing segment C (24) being at least partially made of electrically conducting material (22, 25),
an electronics module (27) arranged within housing segment A (21), comprising:
an electric circuit (40) for receiving and further processing electrical signals, and
at least one first and one second electrical conductor (42, 43) for measuring electrical potentials and/or for delivering electric pulses, each conductor having a conductor start and a conductor end, and each
conductor start being connected to the electric circuit (40);
a battery module (26), the battery module comprising a battery housing formed by housing segment C (24),
the electromedical implant (20) comprising an electrical feedthrough (23), the electrical feedthrough (23) being least partially made of electrically insulating material, and housing segment B being formed by portions of the feedthrough (23),
the electronics module (27) and the battery module (26) being electrically connected via the feedthrough (23), and
housing segment A (21) and housing segment C (24) being electrically insulated from one another by the feedthrough (23), and
the conductor end of the first electrical conductor (42) being electrically connected via the feedthrough (23) to the portion (25) of the housing segment C which is made of electrically conducting material,
**characterized in that**
the conductor end of the second electrical conductor (43) is electrically connected to the portion (22) of the housing segment A which is made of electrically conducting material.

2. The electromedical implant according to claim 1, wherein the electromedical implant (20) is designed as a neurostimulation implant in the form of a spinal cord stimulation device, a vagus nerve stimulation device, or a brain stimulation device, or as a muscle stimulation device, or as a cardiac implant in the form of a sensor, a monitoring device, a pacemaker device, a pacemaker device having an integrated defibrillator function or a pacemaker device without intracardiac electrodes.

3. The electromedical implant according to claim 1 or 2, wherein housing segment A (21) and/or housing segment C (24) comprise a coating made of electrically insulating material, housing segment A (21) and/or housing segment C (24) comprising at least one region having no coating made of electrically insulating material.

4. The electromedical implant according to at least one of the preceding claims, wherein the electrical feedthrough (23) is made of a multilayer technology material.

5. The electromedical implant according to at least one of the preceding claims, wherein the electromedical implant (20) comprises at least one active or passive component (54), which is integrated in the feedthrough (23) and/or joined to the feedthrough (23).

6. The electromedical implant according to at least one of the preceding claims, wherein the electromedical implant (20) comprises an antenna, which is integrated in the feedthrough (23) and/or joined to the feedthrough (23).

7. The electromedical implant according to at least one of the preceding claims, wherein housing segment A (21) is joined to housing segment B and/or housing segment B is joined to housing segment C (24) by way of a low-temperature solder j oint, a laser solder j oint, a cold weld j oint, a friction weld j oint, a fusion weld joint, an ultrasonic weld joint or an adhesive joint.

8. The electromedical implant according to at least one of the preceding claims, wherein the electrical feedthrough (23) has a composition comprising multiple layers (60), the layers being alternately made of electrically insulating material (61) and electrically conducting material (62).

9. A method for producing an electromedical implant (20), comprising the following steps:
- providing an electronics module (27), comprising an electric circuit (40) for receiving and further processing electrical signals, including at least two electrical conductors (42, 43) for measuring electrical potentials;
- providing a battery module (26), an electrical feedthrough (23), wherein the feedthrough (23) is at least partially made of electrically insulating material, a housing shell A (21) and a housing shell C (24), wherein housing shells A (21) and C (24) are at least partially made of electrically conducting material (22, 25);
- arranging the feedthrough (23) between the electronics module (27) and the battery module (26);
- establishing an electrical connection (28a, 28b) between the electronics module (27) and the battery module (26) via the feedthrough (23);
- establishing an electrical connection between the electric circuit (40) and the portion (22) of housing shell A (21) which is made of electrically conducting material;
- establishing an electrical connection between the electric circuit (40) and the portion (25) of housing shell C (24) which is made of electrically conducting material via the feedthrough (23);
- attaching housing shell A (21) on the side of the feedthrough (23) located toward the electronics module (27) so that the electronics module (27) is arranged within housing shell A (21) and is hermetically sealed; and
- attaching housing shell C (24) on the side of the feedthrough (23) located toward the battery module (26) so that the battery module (26) is arranged within housing shell C (24) and is hermetically sealed so that
- housing shell A (21) and housing shell C (24) are electrically insulated from one another by the feedthrough (23).

10. The method for producing an electromedical implant (20) according to claim 9, wherein the electromedical implant (20) is designed as a cardiac implant in the form of a sensor, a monitoring device, a pacemaker device, a pacemaker device having an integrated defibrillator function or a pacemaker device without intracardiac electrodes.

11. The method for producing an electromedical implant (20) according to claim 9 or 10, comprising the following further step:
- coating housing shell A (21) and/or housing shell C (24) with electrically insulating material, a region of housing shell A (21) and/or of housing shell C (24) not being coated.

12. The method for producing an electromedical implant (20) according to at least one of claims 9 to 11, wherein the attaching of housing shell A (21) and/or housing shell C (24) takes place by way of low-temperature soldering, laser soldering, cold welding, friction welding, fusion welding, ultrasonic welding or gluing.

13. The method for producing an electromedical implant (20) according to at least one of claims 9 to 12, comprising the following further step:
- integrating at least one passive or active component (54) and/or an antenna in the feedthrough (23).

14. The method for producing an electromedical implant (20) according to at least one of claims 9 to 13, wherein the electrical feedthrough (23) has a composition comprising multiple layers, the layers being alternately made of electrically insulating material (61) and electrically conducting material (62).

15. The method for producing an electromedical implant (20) according to at least one of claims 9 to 14, wherein housing shell A (21) and/or housing shell C (24) are composed of two joined half shells (72, 73).

## Revendications

1. Implant électromédical (20) comprenant
un boitier, présentant
trois segments de boitier A (21), B et C (24), où
le segment de boitier A (21) est relié avec le segment de boitier B, et le segment de boitier B est relié avec le segment de boitier C(24), de sorte que le segment de boitier B est disposé entre les segments de boitier A (21) et C (24), où le segment de boitier A (21) ainsi que le segment de boitier C (24) sont constitués au moins partiellement d'un matériau (22, 25) électriquement conducteur,
un module électronique (27) qui est disposé à l'intérieur du segment de boitier A (21), comprenant
un circuit électrique (40) électrique pour la réception et le traitement ultérieur de signaux électriques, et
au moins un premier et un deuxième conducteur électrique (42, 43) pour la mesure de potentiels électriques et/ou la délivrance d'impulsions électriques, où chaque conducteur présente respectivement un début de conducteur et une terminaison de conducteur et chaque début de conducteur est relié avec le circuit électrique (40) électrique,
un module de batterie (26), où le module de batterie présente un boitier de batterie, lequel est formé par le segment de boitier C (24),
où l'implant électromédical (20) présente une traversée électrique (23), où la traversée électrique (23) est constituée au moins partiellement d'un matériau isolant électriquement et le segment de boitier B est formé par des parties de la traversée (23),
où le module électrique (27) et le module de batterie (26) sont reliés électriquement par le biais de la traversée (23), et
le segment de boitier A (21) et le segment de boitier C (24) sont isolés électriquement l'un de l'autre par la traversée (23),
et où la terminaison de conducteur du premier conducteur électrique (42) est reliée électriquement avec la partie (25) du segment de boitier C constituée du matériau conducteur électriquement par le biais de la traversée (23), **caractérisé en ce que**
la terminaison de conducteur du deuxième conducteur électrique (43) est reliée électriquement avec la partie (22) du segment de boitier A constituée du matériau électriquement conducteur.

2. Implant électromédical selon la revendication 1, où l'implant électromédical (20) est conçu en tant qu'un implant de neurostimulation sous forme d'un appareil de stimulation de moelle épinière, d'appareil de stimulation du nerf vagal ou d'appareil de stimulation cervical, ou est conçu en tant qu'appareil de stimulation musculaire, ou est conçu en tant qu'implant cardiaque sous forme d'un capteur, d'un appareil de surveillance, d'un appareil de stimulation, d'un appareil stimulateur avec une fonction de défibrillation intégrée ou d'un appareil stimulateur sans électrodes intra cardiales.

3. Implant électromédical selon la revendication 1 ou la revendication 2, dans lequel le segment de boitier A (21) et/ou le segment de boitier C (24) présentent un revêtement à base d'un matériau isolant électriquement, où le segment de boitier A (21) et/ou le segment de boitier C (24) présentent au moins une région sans revêtement à base de matériau isolant électriquement.

4. Implant électromédical selon au moins une des revendications précédentes, dans lequel la traversée (23) électrique est constituée d'un matériau de technologie multicouches.

5. Implant électromédical selon au moins une des revendications précédentes, où l'implant médical (20) présente au moins un composant actif ou passif (54) qui est intégré dans la traversée (23) et/ou est relié avec la traversée (23).

6. Implant électromédical selon au moins une des revendications précédentes, où l'implant médical (20) présente une antenne qui est intégrée dans la traversée (23), et/ou est reliée avec la traversée (23).

7. Implant électromédical selon au moins une des revendications précédentes, dans lequel le segment de boitier A (21) est relié avec le segment de boitier B et/ou le segment de boitier B est relié au segment de boitier C (24) au moyen d'un soudage à basse température, d'un soudage laser, d'une liaison par soudage à froid, d'une liaison par soudage en friction, par une liaison par soudage de fusion, par une liaison par soudage aux ultrasons ou une liaison par collage.

8. Implant électromédical selon au moins une des revendications précédentes, dans lequel la traversée (23) électrique présente une construction en plusieurs couches, (60), où les couches sont constituées alternativement de matériau électriquement isolant (61) et de matériau électriquement conducteur (62).

9. Procédé de fabrication d'un implant électromédical (20) comprenant les étapes
- de fourniture d'un module électronique (27) comprenant un circuit électrique (40) pour la réception et le traitement ultérieur de signaux électriques avec au moins deux conducteurs électriques (42, 43) pour la mesure de potentiels électriques,
- de fourniture d'un module de batterie (26), d'une traversée électrique (23), où la traversée (23) est constituée au moins partiellement d'un matériau isolant électriquement, d'une coque de boitier A (21) et d'une coque de boitier C (24), où les coques de boitier A (21) et C (24) sont constituées au moins partiellement d'un matériau conducteur électriquement (22, 25),
- de disposition de la traversée (23) entre le module électronique (27) et le module de batterie (26),
- d'établissement d'une connexion électrique (28a, 28b) entre le module électronique (27) et le module de batterie (26) par le biais de la traversée (23),
- d'établissement d'une connexion électrique entre le circuit électrique (40) et la partie (22) de la coque de boitier A (21) constituée du matériau conducteur électriquement,
- d'établissement d'une connexion électrique entre le circuit électrique (40) et la partie (25) de la coque de boitier C (24) constituée du matériau conducteur électriquement par le biais de la traversée (23),
- de rattachement de la coque de boitier A (21) au côté de la traversée (23) situé vers le module électronique (27) de sorte que le module électronique (27) est disposé à l'intérieur de la coque de boitier A (21) et y est raccordé hermétiquement, et
- de rattachement de la coque de boitier C (24) au côté de la traversée (23) situé vers le module de batterie (26) de sorte que le module de batterie (26) est disposé à l'intérieur de la coque de boitier C (24) et y est raccordé hermétiquement, de sorte que
- la coque de boitier A (21) et la coque de boitier C (24) sont isolées électriquement l'une de l'autre par la traversée (23).

10. Procédé de fabrication d'un implant électromédical (20) selon la revendication 9, où l'implant électromédical (20) est conçu en tant qu'un implant cardiaque sous forme d'un capteur, d'un appareil de surveillance, d'un appareil de stimulation, d'un appareil stimulateur avec une fonction de défibrillation intégrée ou d'un appareil stimulateur sans électrodes intra cardiales.

11. Procédé de fabrication d'un implant électromédical (20) selon la revendication 9 ou la revendication 10, comprenant l'étape supplémentaire :
- de revêtement de la coque de boitier A (21) et/ou de la coque de boitier C (24) avec un matériau isolant électriquement, où une région de la coque de boitier A (21) et/ou de la coque de boitier C (24) n'est pas revêtue.

12. Procédé de fabrication d'un implant électromédical (20) selon au moins une des revendications 9 à 11, dans lequel le rattachement de la coque de boitier A (21) et/ou de la coque de boitier C (24) a lieu par un soudage à basse température, un soudage laser, un soudage à froid, un soudage en friction, un soudage de fusion, un soudage aux ultrasons ou un collage.

13. Procédé de fabrication d'un implant électromédical (20) selon au moins une des revendications 9 à 12, comprenant l'étape supplémentaire :
- l'intégration d'au moins un composant (54) passif ou actif et/ou d'une antenne dans la traversée (23).

14. Procédé de fabrication d'un implant électromédical (20) selon au moins une des revendications 9 à 13, dans lequel la traversée (23) électrique présente une construction en plusieurs couches, où les couches sont constituées alternativement d'un matériau isolant (61) électriquement et d'un matériau conducteur (62) électriquement.

15. Procédé de fabrication d'un implant électromédical (20) selon au moins une des revendications 9 à 14, dans lequel la coque de boitier A (21), et/ou la coque de boitier C (24) est construite à base de deux demies coques (72, 73) assemblées.
